# EUROPÄISCHE PATENTANMELDUNG

(11) **EP 4 276 852 A2**
(43) Veröffentlichungstag der Anmeldung: **15.11.2023**
(21) Anmeldenummer: 23189065.8
(22) Anmeldetag: 24.02.2020
(51) Int. Cl.: G16H 50/70

(54) **SYSTEM ZUR AKUSTISCHEN ERKENNUNG VON OBSTRUKTIONSARTEN BEI DER SCHLAFAPNOE UND ENTSPRECHENDES VERFAHREN**

(30) Priorität: 07.03.2019 DE 102019105762
(62) Teilanmeldung aus: 20715275.2
(71) Anmelder: Diametos GmbH, 14469 Potsdam (DE)
(72) Erfinder: Janott, Christoph, 10589 Berlin (DE)
(74) Vertreter: Farago-Schauer, Peter Andreas

(57) **Zusammenfassung**

Die vorliegende Erfindung betrifft ein Klassifikationssystem (1) zu einer mikroprozessorgestützten Erkennung von Obstruktionsarten (01-04) bei einer Schlafapnoe durch entsprechende Klassifikation eines zu untersuchenden Schnarchgeräuschsignals (Au), umfassend:
a) eine Eingangsschnittstelle für das jeweilige Schnarchgeräuschsignal (Au);
b) einen ersten Klassifikator (K1), der so trainierbar ist, dass er zu einem jeweiligen Schnarchgeräuschsignal (Au) die jeweils wahrscheinlichste Schnarchgeräuschentstehungsart (S1-S4) erkennt und ausgibt;
c) einen zweiten Klassifikator (K2), der so trainierbar ist, dass er zu einem jeweiligen Schnarchgeräuschsignal (Au) die jeweils wahrscheinlichste Mundstellung (M1-M2) erkennt und ausgibt; und
d) einen dritten Klassifikator (K3), der ausgebildet ist, in einem Trainingsmodus und/oder in einem Erkennungsmodus die wahrscheinlichste Obstruktionsart (01-04) aus der bestimmten Schnarchgeräuschentstehungsart (S1-S4), der bestimmten Mundstellung (M1-M2) und in Abhängigkeit einem oder mehreren weiteren Patientenparametern, die über eine dafür vorbestimmte Eingabeschnittstelle erfasst werden, zu bestimmen.

## Beschreibung

Die vorliegende Erfindung betrifft ein System zu einer mikroprozessorgestützten Erkennung von Obstruktionsarten bei einer Schlafapnoe durch eine entsprechende Klassifikation eines zu untersuchenden Schnarchgeräuschsignals.

Als Schnarchen bezeichnet man nächtliche Atemgeräusche, die durch Vibrationen von Weichgewebe in den oberen Atemwegen entstehen. Es existieren unterschiedliche Definitionen des Begriffs Schnarchen, wobei in vielen Definitionen das Schnarchen eine durch einen Luftstrom verursachte Vibration des Gewebes der oberen Atemwege ist und dabei einen daraus resultierenden tonalen Anteil im entstehenden Schnachgeräusch erzeugt. Eine Abgrenzung zwischen "Schnarchen" und "lautem Atmen" ist jedoch nicht eindeutig definiert. Im Folgenden kann der Begriff "Schnarchen" als generelles Geräusch verstanden werden, das auch Atemgeräusche ohne signifikanten tonalen Anteil enthalten kann.

Unter einem Schnarchgeräuschsignal wird ein akustisches Signal verstanden, das beispielsweise von einem Mikrofon aufgenommen und in ein elektrisches Signal umgewandelt worden ist. Das Schnarchgeräuschsignal kann dabei auch eine zusätzliche Information als Indikatoren oder Labels umfassen oder beigeordnet haben, die eine oder mehrere weitere Informationen zum akustischen Schnarchgeräuschsignal übermitteln, wie beispielsweise einen Schnarchgeräuschentstehungsort, eine Schnarchgeräuschentstehungsart, eine Mundstellung, eine Uhrzeit, einen Patientennamen, ein Patientengewicht, eine Schlafposition.

Unter einer obstruktiven Schlafapnoe bezeichnet man eine Erkrankung, bei der nächtliche Atemaussetzer aufgrund von Verschlüssen bzw. Obstruktionen der oberen Atemwege als sogenannte Atemwegsobstruktionen auftreten. Je nach Anzahl der obstruktiven Atemaussetzer pro Stunde werden verschiedene Schweregrade der obstruktiven Schlafapnoe unterschieden. Das Schnarchen ist für die obstruktiven Schlafapnoe ein häufiges Begleitsymptom. Das Schnarchen und die obstruktive Schlafapnoe sind schlafbezogene Atmungsstörungen. Im Weiteren wird die obstruktive Schlafapnoe auch nur kurz als Schlafapnoe bezeichnet.

Die Schnarchgeräusche und Atemwegsobstruktionen entstehen an unterschiedlichen Orten der oberen Atemwege und in unterschiedlichen Arten. Die unterschiedlichen Arten können dabei durch eine jeweilige Orientierung und Form der Vibration oder Verengung bestimmt sein, die beispielsweise zirkulär oder lateral schlitzförmig sein kann. Dementsprechend gibt es unterschiedliche Schnarchgeräuschentstehungsarten, die mit den unterschiedlichen Obstruktionsorten und Obstruktionsarten anatomisch in Verbindung stehen. In anderen Worten ist die Schnarchgeräuschentstehungsart definiert durch den Schnarchgeräuschentstehungsort, die Orientierung und Form der Vibration, oder eine Kombination daraus. Analog wird im Folgenden die Obstruktionsart durch den Ort der Obstruktion, die Orientierung der Obstruktion oder eine Kombination daraus definiert. Die unterschiedlichen Schnarchgeräuschentstehungsarten lassen sich beispielsweise klassifizieren, wie folgt, verursacht durch:
A) eine anterior-posteriore Vibration des weichen Gaumens;
C) eine zirkuläre Verengung der Atemwege in Velopharynx oder Oropharynx;
L) eine laterale Vibration von Weichgewebe im Bereich des Oropharynx;
T) eine Verengung im Bereich des Zungengrundes; und/oder
E) eine Vibration oder Verengung im Bereich des Kehldeckels.

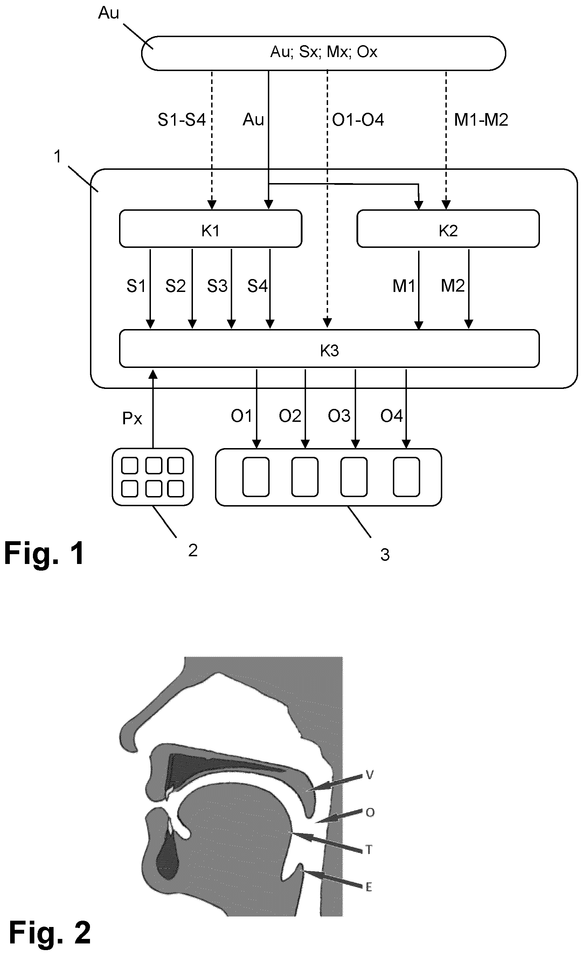

Im Rachenbereich (Pharynx) kann das Schnarchgeräusch zum Beispiel im Bereich des Weichgaumens mit dem Zäpfchen, im Bereich der Gaumenmandeln, im Bereich des Zungengrundes sowie auf Höhe des Kehldeckels erzeugt werden. In der Wissenschaft sind dazu zahlreiche Klassifikationssysteme beschrieben worden, mit dem Ziel, eine standardisierte Bezeichnung von Engstellen und der Obstruktionsarten im klinischen Alltag zu schaffen. Fig. 2 zeigt ein Schnittbild einer Seitenansicht eines Patientenkopfes, das die Schnarchentstehungsorte in einem Hals-Nasen-Rachenraum mit den Bereichen Velopharynx (V), Oropharynx (O), Zungengrund (T) und Kehldeckel (E) zeigt.

Eine Diagnostik schlafbezogener Atmungsstörungen beinhaltet nach den meisten medizinischen Leitlinien eine Messung einer Häufigkeit eines Auftretens von Atemaussetzern (Apnoen) durch Obstruktionen und Atembeeinträchtigungen durch Atemwegsverengungen (Hypopnoen) im natürlichen Schlaf, wobei die Messungen sowohl im Schlaflabor als Polysomnographie als auch im häuslichen Umfeld als Polygraphie und als kardiorespiratorisches Screening durchgeführt werden. Diese Messungen liefern aber keine eindeutigen Informationen über einen Ort der Engstelle oder der Obstruktion. Abhängig von Ort und Form der Verengung oder der Obstruktion können dementsprechende unterschiedliche therapeutische Maßnahmen vorgenommen werden. Neben der Kenntnis einer Schwere der obstruktiven Schlafapnoe, die durch die Häufigkeit und eine Länge der Atemaussetzer bestimmt ist, ist für eine gezielte Therapie der obstruktiven Schlafapnoe die Kenntnis der jeweiligen Obstruktionsart sehr wichtig. Ebenso ist für eine gezielte Behandlung des Schnarchens die Schnarchgeräuschentstehungsart sehr wichtig.

Eine bekannte Lösung zur Bestimmung des Schnarchgeräuschentstehungsortes und des Obstruktionsortes ist beispielsweise die nächtliche Manometrie, bei der ein dünner Katheter von wenigen Millimetern Durchmesser, der mit mehreren, in Reihe angeordneten Sensoren ausgestattet ist, über die Nase in die oberen Atemwege des Patienten eingeführt wird. So können während der Nacht die Druckverhältnisse an mehreren Positionen der oberen Atemwege während des natürlichen Schlafes gemessen werden. Ein Vorteil dieser Methode ist eine kontinuierliche Messung während des natürlichen Schlafs. Ein Nachteil dieses Verfahrens ist, dass nicht jeder Patient einen Katheter für eine gesamte Nacht in den oberen Atemwegen toleriert. Auch kann über die Art und Form der Obstruktion keine Aussage getroffen werden.

Eine weitere bekannte Methode zur Bestimmung der Obstruktionsorte und der Obstruktionsarten bei Patienten ist die medikamenteninduzierte Schlafvideoendoskopie. Bei dieser Methode wird der Patient durch einen Anästhesisten sediert, um einen künstlichen Schlafzustand herzustellen. Dann erfolgt mittels eines flexiblen Endoskops durch die Nase die Beobachtung der oberen Atemwege. So können Ort, Form und Art der Obstruktionen visuell dargestellt und bestimmt werden. Nachteile dieses Verfahrens sind eine Belastung des Patienten mit sedierenden Medikamenten sowie ein hoher personeller und apparativer Aufwand und hohe damit verbundene Kosten. Während der medikamenteninduzierten Schlafvideoendoskopie sind oft Audioaufnahmen der Schnarchgeräusche zeitgleich mit Videoaufnahmen der Endoskopieuntersuchungen erstellt worden, aus welchen im Nachhinein die Schnarchgeräuschsignale mit Informationen zu den jeweiligen Schnarchgeräuschentstehungsarten und/oder den jeweiligen Obstruktionsarten extrahiert werden können. Zwischen Schnarchgeräuschentstehungsart und Obstruktionsart besteht eine gewisse Korrelation, allerdings kann nicht mit ausreichender Sicherheit von der Schnarchgeräuschentstehungsart auf die Obstruktionsart geschlossen werden. Auch treten Obstruktionen und Schnarchgeräusche in der überwiegenden Anzahl der Fälle in einem gewissen zeitlichen Zusammenhang, aber nicht zeitsynchron auf.

Bekannt ist, dass die Mundstellung während des Schlafs einen signifikanten Einfluss auf die Schlafqualität und andere Krankheiten, wie beispielsweise Zahnkrankeiten und auf eine Wahrscheinlichkeit eines Auftretens und die Art einer Atemwegsobstruktion hat. Die Mundstellung wird im Wesentlichen durch die Position des Unterkiefers und der Lippen bestimmt. Im einfachsten Fall werden zwei Zustände unterschieden: Mund geschlossen (z.B. Lippenschluss) und Mund geöffnet. Alternativ lässt sich die Mundstellung mit differenzierteren Definitionen genauer beschreiben.

Während einer medikamenteninduzierten Schlafvideoendoskopie werden, zum Beispiel durch manuelle Bewegung des Unterkiefers des Patienten durch den Untersucher, offene und geschlossene Mundstellungen forciert und der Einfluss der Mundstellung auf die Obstruktionsart dadurch untersucht. Aus den Audioaufnahmen und den endoskopischen Videoaufnahmen einer Schlafvideoendoskopie ist die Mundstellung zum Zeitpunkt des Auftretens von Schnarchgeräuschen jedoch nicht erkennbar. Informationen zur Mundstellung zum Zeitpunkt des Auftretens von Schnarchgeräuschen während Schlafvideoendoskopieuntersuchungen sind auch nicht in strukturierter Form vorhanden.

Zu einer Bestimmung der Mundstellung während des natürlichen Schlafes sind verschiedene beispielsweise videobasierte Verfahren bekannt. Dabei sind eine Vielzahl jeweiliger Schnarchgeräuschsignale aufgenommen worden, zu denen die jeweilige Mundstellung als zusätzliche Information extrahiert werden kann, die als ein Indikator bestimmt und festgehalten wird. Hierbei ist zu bemerken, dass für die Bestimmung der Mundstellung eine videobasierte Bestimmung zeitlich sehr aufwendig ist und eine dazu alternative Bestimmung über Sensoren am Kopf des Patienten für eine Schlafqualität störend sind. So gewonnene wiederum weitere Schnarchgeräuschsignale mit den jeweils dazu bestimmten Mundstellungen sind vorhanden und entsprechend auch wertvoll.

WO2017/009081A1 offenbart ein Gerät und ein Verfahren zur Schnarchgeräuschidentifikation in oberen Atemwegen, indem durch einen Tubus mit bevorzugt zwei versetzen Mikrofonsensoren geatmet wird. Duch Sensorsignale der zwei Mikrofonsensoren lassen sich während einer Inhalation und Exhalation Obstruktionen der oberen Atemwege erkennen.

WO2012/155257A1 offenbart ein Gerät und Verfahren zur Diagnose von Atemwegsgeräuschen und Rhythmen, wobei während des Atmens oder Schlafs ein Mikrofonsensor vor der Nase des Patienten platziert ist.

DE102006008844A1 offenbart ein Verfahren zur Detektion von Atemwegsgeräuschen, wobei ein Sensor vor einem Nasenloch oder darin eingeführt platziert und dessen Sensorsignal entsprechend ausgewertet wird.

Zur Klarheit sei an dieser Stelle angemerkt, dass das jeweilige Schnarchgeräuschsignal die eine oder die mehreren zusätzlichen Information/en umfassen kann oder beigeordnet haben kann, wobei die eine oder die mehreren zusätzlichen Information/en als Indikatoren oder Labels mit dem akustischen Schnarchgeräuschsignal verknüpft sind. Die eine oder die mehreren zusätzlichen Information/en oder Indikatoren sind beispielsweise der Schnarchgeräuschentstehungsort, die Schnarchgeräuschentstehungsart, die Mundstellung und/oder weitere zusätzliche Patientenparameter, wie oben angegeben. Die Indikatoren, die oft auch als Labels bezeichnet werden, können dabei im Schnarchgeräuschsignal selbst beispielsweise aufmoduliert oder kodiert enthalten oder auf einer zweiten Signalspur oder Datei aufgezeichnet sein, oder sie können schriftlich festgehalten sein.

Die Aufgabe der Erfindung, um die Nachteile aus dem Stand der Technik zu beseitigen, besteht daher in der Bereitstellung eines Systems zu einer automatischen und möglichst signifikanten Erkennung einer jeweils wahrscheinlichsten Obstruktionsart aus einem zu untersuchenden Schnarchgeräuschsignal.

Die vorstehende Aufgabe wird von einer Vorrichtung gemäß den Merkmalen des unabhängigen Anspruchs 1 gelöst. Weitere vorteilhafte Ausführungsformen der Erfindung sind in den abhängigen Ansprüchen angegeben.

Erfindungsgemäß wird ein Klassifikationssystem zu einer mikroprozessorgestützten Erkennung von Obstruktionsarten bei einer Schlafapnoe durch entsprechende Klassifikation eines zu untersuchenden Schnarchgeräuschsignals vorgestellt, das Folgendes umfasst:
- eine Eingangsschnittstelle für das jeweilige Schnarchgeräuschsignal;
- einen ersten Klassifikator, der ausgebildet ist, bei Eingabe einer ersten Vielzahl der Schnarchgeräuschsignale mit einer jeweils dazugehörigen Schnarchgeräuschentstehungsart in einem Trainingsmodus so zu lernen, dass er in einem Erkennungsmodus zu dem jeweiligen Schnarchgeräuschsignal aus einer Gruppe von vorbestimmten Schnarchgeräuschentstehungsarten die jeweils wahrscheinlichste Schnarchgeräuschentstehungsart erkennt und ausgibt;
- einen zweiten Klassifikator, der ausgebildet ist, bei Eingabe einer zweiten Vielzahl der Schnarchgeräuschsignale mit einer jeweils dazugehörigen Mundstellung im dazugehörigen Trainingsmodus so zu lernen, dass er im Erkennungsmodus zu dem jeweiligen Schnarchgeräuschsignal aus einer Gruppe von vorbestimmten Mundstellungen die jeweils wahrscheinlichste Mundstellung erkennt und ausgibt;
- einen dritten Klassifikator, der ausgebildet ist, in einem Erkennungsmodus bei Eingabe der vom ersten Klassifikator bestimmten Schnarchgeräuschentstehungsart, der vom zweiten Klassifikator bestimmten Mundstellung aus einer Gruppe von vorbestimmten Obstruktionsarten die wahrscheinlichste Obstruktionsart zu erkennen und als Obstruktionsartsignal auszugeben; und
- eine Ausgabeschnittstelle für das Obstruktionsartsignal zu einer Anzeige.

Bevorzugt ist der dritte Klassifikator auch ausgebildet, in einem Trainingsmodus bei Eingabe der vom ersten Klassifikator bestimmten Schnarchgeräuschentstehungsart, der vom zweiten Klassifikator bestimmten Mundstellung und einer Obstruktionsart so zu lernen, dass er im Erkennungsmodus zu der jeweiligen Schnarchentstehungsart und Mundstellung die beim Trainieren eingegebene Obstruktionsart als die wahrscheinlichste Obstruktionsart erkennt. Zur Klarheit sei gesagt, dass dem Fachmann bekannt ist, was bei Klassifikatoren unter dem Lernen zu verstehen ist.

Die mit der Erfindung erzielten Vorteile bestehen insbesondere darin, dass zum Trainieren des ersten und des zweiten Klassifikators eine Vielzahl der Schnarchgeräuschsignale verwendbar sind, die nur entweder die Schnarchgeräuschentstehungsart oder die Mundstellung als eine zusätzliche Information zu einer rein akustischen Komponente des Schnarchgeräuschsignals enthalten. Die Information kann generell als Indikator oder Label im Schnarchgeräuschsignal selbst codiert oder aufmoduliert enthalten sein, auf einer separaten Signalspur oder in einer separaten Datei aufgezeichnet enthalten oder dem Schnarchgeräuschsignal beigeordnet sein, beispielsweise als schriftliches Label. So kann der erste Klassifikator aus einer bereits im Stand der Technik vorhandenen großen Menge der Schnarchgeräuschsignale, die nur die Schnarchgeräuschentstehungsart als Label umfassen, trainiert werden, ohne den zweiten Klassifikator und/oder den dritten Klassifikator dabei falsch zu trainieren. Ebenso kann der zweite Klassifikator aus einer im Stand der Technik bereits vorhandenen anderen großen Menge der Schnarchgeräuschsignale, die nur die Mundstellung als Label umfassen, trainiert werden, ohne den ersten Klassifikator und/oder den dritten Klassifikator dabei falsch zu trainieren. Das erfindungsgemäße Klassifikationssystem erfordert also nicht eine ganz neue Erfassung der Schnarchgeräuschsignale, die in Verbindung mit den beiden Indikatoren Schnarchgeräuschentstehungsart und Mundstellung zusammen erfasst werden. Indem durch die vorliegende Erfindung die schon existierenden Schnarchgeräuschsignale mit nur dem einem oder anderen Indikator zum Trainieren des ersten und des zweiten Klassifikators verwendbar sind, entsteht ein großer Aufwands- und Kostenvorteil; und dennoch werden bei der Erkennung der Obstruktionsart aus dem zu untersuchenden Schnarchgeräuschsignal beide Indikatoren Schnarchgeräuschentstehungsart und Mundstellung mit berücksichtigt. Indem die beiden Indikatoren Schnarchgeräuschentstehungsart und Mundstellung bei der Erkennung der Obstruktionsart berücksichtigt werden, wird die Genauigkeit einer richtigen Identifikation der jeweiligen Obstruktionsart deutlich erhöht und/oder eine falsche Erkennung unwahrscheinlicher, im Vergleich zu einer Erkennung der Obstruktionsart aus dem zu untersuchenden Schnarchgeräuschsignal mit nur dem Indikator Schnarchgeräuschentstehungsart.

Bevorzugt sind der erste Klassifikator und der zweite Klassifikator so ausgebildet, dass das jeweilige Trainieren des ersten und des zweiten Klassifikators mit einer Vielzahl der Schnarchgeräuschsignale separat voneinander vorgenommen werden kann, wobei der erste Klassifikator unabhängig von der Mundstellung und der zweite Klassifikator unabhängig von der Schnarchgeräuschentstehungsart trainierbar sind. Bevorzugt kann das jeweilige Trainieren des ersten Klassifikators dabei zeitversetzt zum jeweiligen Trainieren des zweiten Klassifikators erfolgen. Alternativ bevorzugt kann das jeweilige Trainieren des ersten Klassifikators auch zeitgleich zum jeweiligen Trainieren des zweiten Klassifikators erfolgen. Zur Klarheit kann die Vielzahl der Schnarchgeräuschsignale eine Menge an Schnarchgeräuschsignalen umfassen, denen als Indikator die Schnarchgeräuschentstehungsart und keine Mundstellung zugeordnet sind, und eine weitere Menge an Schnarchgeräuschsignalen umfassen, denen als Indikator die Mundstellung und keine Schnarchgeräuschentstehungsart zugeordnet sind.

Zur Klarheit sind die Bezeichnungen Indikator und Label hierin Synonyme zueinander. Das Trainieren des Klassifikators kann auch als Lernen bezeichnet werden. Unter dem Trainieren oder dem Trainiermodus des jeweiligen Klassifikators mit dem jeweiligen Schnarchgeräuschsignal mit dem mindestens einen Indikator wird verstanden, dass der jeweilige Klassifikator sich dabei so verändert, dass er im Erkennungsmodus den mindestens einen Indikator aus dem Schnarchgeräuschsignal besser erkennt, bevorzugt im Durchschnitt bei vielen trainierten Schnarchgeräuschsignalen mit jeweiligen Indikatoren, wie der Fachmann weiß. Dem Fachmann ist klar, dass je mehr verschiedene Schnarchgeräuschsignale zum Trainieren des jeweiligen Klassifikators verwendet werden, deren Erkennungsrate steigt.

Natürlich sind der erste und der zweite Klassifikator dabei bevorzugt auch ausgebildet, gemeinsam mit einer weiteren Vielzahl der Schnarchgeräuschsignale trainierbar zu sein, die sowohl die Schnarchgeräuschentstehungsart als auch die Mundstellung als jeweils zugeordnete Indikatoren aufweisen. Auf diese Weise können die erste Vielzahl, die zweite Vielzahl und/oder die weitere Vielzahl der verschiedenen Schnarchgeräuschsignale zum Trainieren des ersten und des zweiten Klassifikators verwendet werden.

Dem Fachmann ist weiterhin klar, dass der erste, der zweite Klassifikator und der dritte Klassifikator technisch jeweils ein Teil nur eines Klassifikators sein können; dabei können das Training und/oder das Erkennen der Schnarchgeräuschentstehungsarten und Mundstellungen und/oder der Obstruktionsarten über Teilklassifikatoren erfolgen. Weiterhin ist dem Fachmann klar, dass die Informationen über Schnarchgeräuschentstehungsart und die Mundstellung alternativ über eine Anzahl von Labels bzw. Indikatoren erfolgen kann, die jeweils eine Kombination aus Schnarchgeräuschentstehungsart und Mundstellung kennzeichnen. Bevorzugt kann das jeweilige Label bzw. der Indikator eine oder mehrere Information zum Schnarchgeräuschsignal aufweisen, wie beispielsweise die Schnarchgeräuschentstehungsart, die Mundstellung, eine Schlafzeit und dergleichen.

Eine verbesserte Erkennung der Schnarchgeräuschentstehungsarten und der Mundstellung aus dem Schnarchgeräuschsignal bewirkt eine daraus resultierende verbesserte Erkennung der entsprechenden Obstruktionsart

Zur Klarheit sei an dieser Stelle bemerkt, dass der erste Klassifikator ein lernender Klassifikator ist, der in einem Trainings- oder Lernmodus jeweils durch die erste Anzahl der Schnarchgeräuschsignale so trainierbar ist, dass er dann in einem Erkennungsmodus aus den jeweiligen Schnarchgeräuschsignalen das jeweilige Schnarchgeräuschentstehungsart mit der jeweils höchsten Wahrscheinlichkeit erkennt. Ebenso ist der zweite Klassifikator ein lernender Klassifikator, der im Trainings- oder Lernmodus jeweils durch die zweite Anzahl der Schnarchgeräuschsignale so trainierbar ist, dass er dann im Erkennungsmodus aus den jeweiligen Schnarchgeräuschsignalen die jeweilige Mundstellung mit der jeweils höchsten Wahrscheinlichkeit erkennt.

Dem Fachmann ist dabei klar, dass der jeweilige Klassifikator bevorzugt auf eine Erkennung vorbestimmter Merkmale optimiert ist.

Das Training und die Erkennung von Schnarchgeräuschsignalen durch die jeweiligen Klassifikatoren können jeweils zeitlich nacheinander, zeitgleich oder zeitlich überlappend erfolgen.

Bevorzugt ist der erste Klassifikator ausgebildet, im Erkennungsmodus die jeweilige Schnarchgeräuschentstehungsart mit einer jeweiligen Wahrscheinlichkeit zu bestimmen, zu indizieren und an den dritten Klassifikator weiterzuleiten. Der dritte Klassifikator wertet dabei die jeweilige Wahrscheinlichkeit der jeweiligen Schnarchgeräuschentstehungsart in Verbindung mit der jeweiligen Mundstellung und ggf. zusätzlichen oder weiteren Schnarch- oder Patientendaten weiter aus. Bevorzugt klassifiziert der erste Klassifikator das Schnarchgeräuschsignal in einen Vektor der Schnarchgeräuschentstehungsarten, wobei jeder Schnarchgeräuschentstehungsart oder Klasse der jeweiligen Schnarchgeräuschentstehungsart als eine jeweilige Wahrscheinlichkeit ausgegeben wird.
Dies erhöht die Auswertemöglichkeit und wahrscheinlichkeitsbedingte Kombinatorik zwischen erkannter Schnarchgeräuschentstehungsart und erkannter Mundstellung für den dritten Klassifikator.

Bevorzugt ist der zweite Klassifikator ausgebildet, im Erkennungsmodus die jeweilige Mundstellung mit einer jeweiligen Wahrscheinlichkeit zu bestimmen, zu indizieren und an den dritten Klassifikator weiterzuleiten. Der dritte Klassifikator wertet dabei die jeweilige Wahrscheinlichkeit der jeweiligen Mundstellung in Verbindung mit der jeweiligen Schnarchgeräuschentstehungsart und ggf. den weiteren Schnarch- oder Patientendaten weiter aus. Bevorzugt klassifiziert der zweite Klassifikator das Schnarchgeräuschsignal in einen Vektor der Mundstellungen, wobei jede Mundstellung oder Klasse der jeweiligen Mundstellung als eine jeweilige Wahrscheinlichkeit ausgegeben wird.

Bevorzugt ist der dritte Klassifikator ausgebildet, im Erkennungsmodus die jeweilige Obstruktionsart mit einer jeweiligen Wahrscheinlichkeit zu bestimmen, zu indizieren und an die Ausgabeschnittstelle weiterzuleiten. Die Ausgabeschnittstelle stellt die jeweilige Obstruktionsart dann einer Anzeige zur Verfügung, dabei kann die Ausgabeschnittstelle eine beliebige der bekannten dafür geeigneten Schnittstellen sein, wie beispielsweise eine elektrische Schnittstelle oder eine drahtlose Funkschnittstelle, beispielsweise zu einem Smartphone oder einer Anzeige an einem PC. Die Ausgabeschnittstelle kann dabei auch über das Internet geleitet sein, so dass die Auswertung und Anzeige an verschiedenen Orten geschieht.

Die einzelnen Bestandteile des beschriebenen Systems können auch räumlich voneinander getrennt angeordnet sein. Bevorzugt werden die entsprechenden Informationen zwischen den Systembestandteilen dann über geeignete Schnittstellen übermittelt, welche beispielsweise elektrische Schnittstellen oder drahtlose Funkschnittstellen sein können. Die Informationen können dabei auch über das Internet geleitet sein.

Bevorzugt ist der dritte Klassifikator ausgebildet, zusätzlich zur jeweiligen Schnarchgeräuschentstehungsart und der jeweiligen Mundstellung die zusätzlichen oder weiteren den Schnarcher betreffenden Schnarch- oder Patientendaten über eine Eingabeschnittstelle zu erfassen und im Trainingsmodus und/oder im Erkennungsmodus bei der Klassifikation der Obstruktionsart zu berücksichtigen. Dabei können die weiteren Schnarch- oder Patientendaten Parameter oder Parametersignale sein, um die jeweils wahrscheinlichste Obstruktionsart noch besser bestimmen zu können. Unter dem Begriff "besser" ist dabei "mit einer höheren Trefferquote" gemeint.

Bevorzugt umfassen die Schnarch- oder Patientendaten dabei mindestens einen der folgenden Parameter: ein Geschlecht, einen Bodymass-Index, einen Apnoe-Hypopnoe-Index, eine Tonsillengröße, eine Zungengröße, einen Friedman-Score, eine Uhrzeit des Schnarchens, eine Schlafdauer und/oder ein Patientengewicht.

Schnarchereignisse und Obstruktionsereignisse können, müssen aber nicht immer zeitgleich auftreten. Zur Klarheit sei daher bemerkt, dass das Label der Obstruktionsart, welches zusammen mit den jeweiligen Informationen über die Schnarchgeräuschentstehungsart und die Mundstellung für das Training des dritten Klassifikators verwendet wird, auch die Obstruktionsart von Obstuktionsereignissen bezeichnen kann, in einem gewissen/engen zeitlichen Zusammenhang, aber nicht genau zeitgleich mit dem jeweiligen Schnarchereignis bei diesem Patienten aufgetreten sind.

Bevorzugt ist der erste Klassifikator auf einem der folgenden Verfahren des maschinellen Lernens bzw. einer Klassifikation basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression. Andere aus dem Stand der Technik bekannte Verfahren sind dabei ebenso denkbar und können hierin Anwendung finden.

Bevorzugt ist der zweite Klassifikator auf einem der folgenden Verfahren des maschinellen Lernens bzw. einer Klassifikation basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression. Andere aus dem Stand der Technik bekannte Verfahren sind dabei ebenso denkbar und können hierin Anwendung finden.

Bevorzugt ist der dritte Klassifikator auf einem der folgenden Verfahren des maschinellen Lernens bzw. einer Klassifikation basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression. Andere aus dem Stand der Technik bekannte Verfahren sind dabei ebenso denkbar und können hierin Anwendung finden.

Denkbar ist dabei auch, dass dem ersten und/oder zweiten Klassifikator die oder ein Teil der zusätzlichen Schnarch- oder Patientendaten zugeführt werden, die der jeweilige erste und/oder zweite Klassifikator bei seiner Klassifikation des Schnarchgeräuschsignals mit auswerten oder berücksichtigen kann. Beispielsweise kann dem jeweiligen Klassifikator ein Merkmal eines Patientengeschlechts eines Bodymass-Index, eines Apnoe-Hypopnoe-Index, einer Tonsillengröße, einer Zungengröße, eines Friedman-Score, einer Uhrzeit des Schnarchens und/oder einer Schlafdauer zugeführt werden.

Alternativ bevorzugt ist der dritte Klassifikator auf einer Matrixwahrscheinlichkeitsberechnung eines ersten Eingangsvektors aus den Schnarchgeräuschentstehungsarten und aus mindestens einem weiteren zweiten Eingangsvektor der Mundstellungen basiert, deren summarische Wahrscheinlichkeiten die verschiedenen Obstruktionsarten mit deren Wahrscheinlichkeiten ergeben.

Zur Klarheit wird bevorzugt unter der ersten Gruppe der Schnarchgeräuschentstehungsarten eine erste Gruppe von ersten Klassen der Schnarchgeräuschentstehungsarten verstanden.

Bevorzugt umfasst die Gruppe der Schnarchgeräuschentstehungsarten folgende Klassen: Velopharynx (V), Oropharynx (O), Zungengrundbereich (T), und/oder Kehldeckelbereich (E). Andere Klassen oder Schnarchgeräuschentstehungsorte oder -arten sind natürlich ebenso denkbar.

Zur Klarheit wird unter der Gruppe von Mundstellungen bevorzugt die Gruppe von Klassen der Mundstellungen verstanden. Bevorzugt umfasst die Gruppe der Mundstellungen die Mundstellungen "Mund offen" und "Mund geschlossen"; andere Mundstellungen und Zwischenstellungen sind natürlich ebenso denkbar.

Zur Klarheit wird bevorzugt unter der Gruppe der Obstruktionsarten die Gruppe von Klassen der Obstruktionsarten verstanden.

Zur Klarheit beschreibt die Art der Schallentstehung dabei neben dem Ort oder anstelle des Ortes der Schallentstehung eine Orientierung und/oder Form der Vibration, die hierin als Schnarchgeräuschentstehungsart bezeichnet wird.

Zur Klarheit werden die Indikatoren, oder auch Label genannt, bevorzugt auf einem objektiven Referenzwert (Ground Truth) basierend ermittelt, bevorzugt ermittelt durch Beobachtung des endoskopischen Bildes einer medikamenteninduzierten Schlafvideoendoskopie durch einen erfahrenen Beobachter zum Zeitpunkt des Auftretens des jeweiligen Schnarchereignisses. Indikatoren oder Label für die Mundstellung werden bevorzugt über die Beobachtung des Patienten während der Untersuchung, Auswertung von Videoaufzeichnungen des Mundbereichs des Patienten während der Untersuchung, oder Aufzeichnung von Sensordaten über den Luftstrom durch Mund und Nase oder andere Sensoren und durch eine Dokumentation der Mundstellung über der Zeit einer Aufnahme des Schnachgeräuschsignals gewonnen.

Aus einer ausreichenden Anzahl von Merkmalsvektoren und Trainingsdaten generiert der jeweilige Klassifizierer, der ein maschineller Klassifizierer ist, mindestens ein Modell. Wird dem Modell ein Merkmalsvektor ohne Label zugeführt, so gibt das Modell einen Ergebniswert aus. Der Ergebniswert enthält eine Information über die wahrscheinlichste Klasse, der das dem Merkmalsvektor zugrundeliegende Schnarchereignis zugehört. In einer alternativen Ausführungsform gibt das Modell zusätzlich eine Information über die Wahrscheinlichkeit aus, mit der das Schnarchereignis der wahrscheinlichsten Klasse zugehört, alternativ darüber hinaus auch die Wahrscheinlichkeiten der Zugehörigkeit zu den anderen Klassen, wie oben beschrieben. Bevorzugt entsprechen die ausgegebenen Klassen den Klassen der für das Training verwendeten Label.

Bevorzugte Ausführungsformen gemäß der vorliegenden Erfindung sind in nachfolgenden Zeichnungen und in einer detaillierten Beschreibung dargestellt, sie sollen aber die vorliegende Erfindung nicht ausschließlich darauf begrenzen.

Es zeigen
- Fig. 1: schematisch ein Klassifikationssystem mit einem ersten und einem zweiten Klassifikator, dem jeweils über eine Eingangsschnittstelle ein Schnarchgeräuschsignal mit einem jeweiligen optionalen Indikator zugeführt wird, wobei die Ausgangssignale des ersten und zweiten Klassifikators einem dritten Klassifikator zur Klassifikation zugeführt werden, die Ausgangssignale des dritten Klassifikators, die Obstruktionsarten darstellen, werden über eine Ausgangsschnittstelle an eine Anzeigeeinheit weitergeleitet und dort angezeigt; Zusätzliche Schnarch- oder Patientendaten können über eine Eingabeschnittstelle eingegeben werden, die dem dritten Klassifikator zur Klassifikation zugeführt werden;
- Fig. 2: ein Schnittbild einer Seitenansicht eines Patientenkopfes, das den Hals-Nasen-Rachenraum mit den Bereichen Velopharynx, Oropharynx, Zungengrund und Kehldeckel zeigt; und

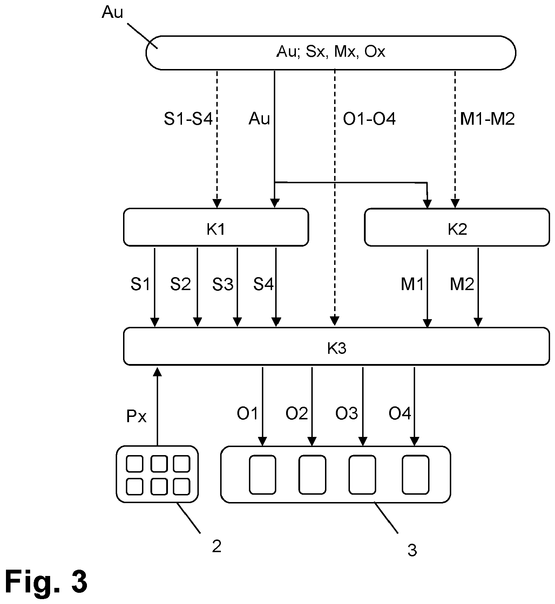

- Fig. 3: ein Signalflussdiagramm eines Verfahrens zur Bestimmung der jeweiligen wahrscheinlichsten Obstruktionsart oder von Wahrscheinlichkeiten der jeweiligen Obstruktionsart aus dem Schnarchgeräuschsignal, das zu einem jeweiligen Trainieren des ersten und zweiten Klassifikators die optionalen Indikatoren mit sich führt.

### Detaillierte Beschreibung von Ausführungsbeispielen

In Fig. 1 ist ein mögliches Ausführungsbeispiel eines Klassifikationssystems 1 zu einer mikroprozessorgestützten Erkennung von Obstruktionsarten 01-04, die bei einer Schlafapnoe auftreten können und vom Klassifikationssystem 1 aus einem zu untersuchenden Schnarchgeräuschsignal Au erkannt werden, schematisch skizziert. Dabei umfasst das Klassifikationssystems 1 folgende Komponenten:
A) eine Eingangsschnittstelle für das jeweilige Schnarchgeräuschsignal Au, die analoge und/oder digitale Eingänge aufweisen kann. Das Schnarchgeräuschsignal Au hat für ein Trainieren des Klassifikationssystems 1 mindestens einen zusätzlichen Indikator oder ein Label, das eine Schnarchgeräuschentstehungsart S1-S4 und/oder eine Mundstellung M1-M2, die dem jeweiligen Schnarchgeräuschsignal Au zugeordnet ist. Bevorzugt weist das Schnarchgeräuschsignal Au auch eine Obstruktionsart 01-04 als Indikator auf, der zum Trainieren des Klassifikationssystems 1 verwendet werden kann. Die Eingangsschnittstelle kann generell auch einen Eingang für eine Tastatur, einen Taster, einen optischen Eingang oder Scanner oder ähnliches aufweisen, um den oder die Indikator/en oder Labels zu erfassen und weiterzuleiten.
B) einen ersten Klassifikator K1, der ausgebildet ist, in einem Trainingsmodus bei Eingabe einer ersten Vielzahl jeweiliger Schnarchgeräuschsignale Au mit einer jeweils dazugehörigen Schnarchgeräuschentstehungsart S1-S4 so zu lernen, dass er in einem Erkennungsmodus zu dem jeweiligen Schnarchgeräuschsignal Au aus einer Gruppe von vorbestimmten Schnarchgeräuschentstehungsarten S1-S4 die jeweils wahrscheinlichste dazugehörige Schnarchgeräuschentstehungsart S1-S4 erkennt und ausgibt. Der erste Klassifikator ist also ein lernender Klassifikator. Zur Klarheit, wenn man im Erkennungsmodus die Schnarchgeräuschsignale Au der Trainingsdaten eingäbe, würden die jeweiligen dazugehörigen Schnarchgeräuschentstehungsarten S1-S4 wieder richtig oder mit höchster Wahrscheinlichkeit ausgegeben, im Mittel zumindest, mit den zuvor beschriebenen bevorzugten Klassifikatoren. Wenn dann im Erkennungsmodus das zu untersuchende Schnarchgeräuschsignal Au eingegeben wird, wird die höchstwahrscheinliche Schnarchgeräuschentstehungsart S1-S4 oder die Schnarchgeräuschentstehungsarten S1-S4 als Wahrscheinlichkeitswerte bestimmt und an einen dritten Klassifikator K3 weitergeleitet.
C) einen zweiten Klassifikator K2, der ausgebildet ist, bei Eingabe einer zweiten Vielzahl der Schnarchgeräuschsignale Au mit einer jeweils dazugehörigen Mundstellung M1-M2 im dazugehörigen Trainingsmodus so zu lernen, dass er im Erkennungsmodus zu dem jeweiligen Schnarchgeräuschsignal Au aus einer Gruppe von vorbestimmten Mundstellungen M1-M2 die jeweils dazugehörige wahrscheinlichste Mundstellung M1-M2 erkennt und ausgibt. Der zweite Klassifikator ist also auch ein lernender Klassifikator. Wenn dann im Erkennungsmodus das zu untersuchende Schnarchgeräuschsignal Au eingegeben wird, wird die höchstwahrscheinliche Mundstellung M1-M2 oder die Mundstellungen M1-M2 als Wahrscheinlichkeitswerte bestimmt und an den dritten Klassifikator K3 weitergeleitet.
D) den dritten Klassifikator K3, der ausgebildet ist, in einem dazugehörigen Erkennungsmodus bei Eingabe der vom ersten Klassifikator K1 bestimmten Schnarchgeräuschentstehungsart S1-S4, der vom zweiten Klassifikator K2 bestimmten Mundstellung M1-M2 aus einer Gruppe von vorbestimmten Obstruktionsarten 01-04 die wahrscheinlichste Obstruktionsart 01-04 zu erkennen und als Obstruktionsartsignal auszugeben.

Bevorzugt ist der dritte Klassifikator K3 ausgebildet, in einem Trainingsmodus bei Eingabe der vom ersten Klassifikator K1 bestimmten Schnarchgeräuschentstehungsart S1-S4, der vom zweiten Klassifikator K2 bestimmten Mundstellung M1-M2 und einer Obstruktionsart 01-04 so zu lernen, dass er im Erkennungsmodus zu der jeweiligen Schnarchentstehungsart S1-S4 und Mundstellung M1-M2 die eingegebene Obstruktionsart 01-04 als die wahrscheinlichste Obstruktionsart 01-04 erkennt.

Wenn im Erkennungsmodus das zu untersuchende Schnarchgeräuschsignal Au eingegeben wird, werden die vom ersten Klassifikator K1 bestimmten Schnarchgeräuschentstehungsarten S1-S4 und die vom zweiten Klassifikator K2 bestimmten Mundstellungen M1-M2 klassifiziert zu der dabei wahrscheinlichsten Obstruktionsart 01-04 oder den Obstruktionsarten 01-04 mit entsprechenden Wahrscheinlichkeitswerten. Der dritte Klassifikator K3 kann auch eine Verknüpfungsmatrix darstellen, die, wie oben beschrieben, eine fest vorgegebene vorbestimmte Wahrscheinlichkeitsberechnung über Eingangsparameter, wie zumindest die Schnarchgeräuschentstehungsarten S1-S4 und den Mundstellungen M1-M2 vornimmt. Dabei kann die Verknüpfungsmatrix durch einen implementierten oder nebengeordneten Lernalgorithmus auch ausgebildet sein, dass die fest vorgegebene vorbestimmte Wahrscheinlichkeitsberechnung in einem Trainingsmodus bevorzugt vor einem Erkennungsmodus oder während eines fortlaufenden Erkennens weiter gelernt werden; und
E) eine Ausgabeschnittstelle 3 für das Obstruktionsartsignal zu einer Anzeige.

Bevorzugt weist das Klassifikationssystem 1 noch eine Eingabeschnittstelle 2 auf, über die zusätzliche Schnarch- und Patientendaten Px eingegeben werden können, die beispielsweise vom dritten Klassifikator K3 bei der Klassifikation der entsprechenden Obstruktionsart 01-04 mitberücksichtigt werden.

Zur Klarheit sei gesagt, dass mit der Schnarchgeräuschentstehungsart S1-S4 oder den Schnarchgeräuschentstehungsarten S1-S4, mit der Mundstellung M1-M2 oder den Mundstellungen M1-M2, und mit der Obstruktionsart 01-04 oder den Obstruktionsarten O1-O4 Signale gemeint sein können, wo sie Signalcharakter besitzen.

Eine Erkennungsgenauigkeit wird bevorzugt mittels annotierter Testdaten ermittelt. Bevorzugt sind die Testdaten ein unabhängiger Teil des Trainingsdatensatzes, welcher jedoch nicht zum Training verwendet wurde.

Bevorzugt ist das Schnarchgeräuschsignal Au ein Signal oder ein Signalvektor, der ein Mikrofon- oder Audiosignal, das das Schnarchgeräuschsignal darstellt, und einen oder mehrere Merkmalssignale umfasst. Dabei kann das Mikrofon- oder Audiosignal, der das Schnarchgeräuschsignal darstellt, in verschiedener Weise vorverarbeitet sein, beispielsweise durch eine Bandpassfilterung oder wie im Stand der Technik bekannt.

Alternativ bevorzugt ist das Schnarchgeräuschsignal Au ein mit einem Merkmalsextraktor aus dem Audiosignal gewonnener Merkmalsvektor, bestehend aus mindestens einem oder mehreren akustischen Merkmalen. Die akustischen Merkmale sind beispielsweise eine Grundfrequenz, ein Energieverhältnis von harmonischen zu nichtharmonischen Anteilen (Harmonic-Noise-Ratio, HNR), Mel-Frequency Cepstral-Koeffizienten (MFCC) und/oder andere. Bevorzugt extrahiert der Merkmalsextraktor statt eines einzelnen Wertes pro Merkmal, welcher einen gesamten Zeitraum eines Schnarchereignisses beschreibt, Informationen über einen zeitlichen Verlauf der akustischen Merkmale, die bevorzugt als statistische Größen dargestellt sind. Die statistischen Größen sind dabei bevorzugt ein Mittelwert, ein Medianwert, eine Standardabweichung und/oder eine Gauß-Verteilung.

Ein zu dem oben beschriebenen Klassifikationssystem passendes Verfahren zu einer mikroprozessorgestützten Erkennung der Obstruktionsarten 01-04 bei der Schlafapnoe durch Klassifikation des aufgenommenen und zu untersuchenden Schnarchgeräuschsignals Au umfasst die folgenden Schritte:
A) Trainieren eines ersten Klassifikators K1 durch Eingabe an dessen Eingang einer ersten Vielzahl an Schnarchgeräuschsignalen Au, denen eine jeweilige Schnarchgeräuschentstehungsart S1-S4 zugeordnet ist, zur Klassifikation und Ausgabe der jeweils wahrscheinlichsten Schnarchgeräuschentstehungsart S1-S4 in einem zugehörigen Erkennungsmodus, wobei die jeweilige Schnarchgeräuschentstehungsart S1-S4 aus einer ersten Gruppe von Klassen der möglichen Schnarchgeräuschentstehungsarten S1-S4 stammt;
B) Trainieren eines zweiten Klassifikators K2 durch Eingabe an dessen Eingang einer zweiten Vielzahl der Schnarchgeräuschsignale Au, denen eine jeweilige Mundstellung M1-M2 zugeordnet ist, zur Klassifikation und Ausgabe der jeweils wahrscheinlichsten Mundstellung M1-M2 im zugehörigen Erkennungsmodus, wobei die jeweilige Mundstellung M1-M2 aus einer zweiten Gruppe von Klassen der möglichen Mundstellungen M1-M2 stammt;
C) Bevorzugt Trainieren oder matrixförmiges Verknüpfen eines dritten Klassifikators K3 durch Eingabe an dessen Eingang der oben bestimmten Schnarchgeräuschentstehungsarten S1-S4 und Mundstellungen M1-M2 zur im zugehörigen Erkennungsmodus Klassifikation und Ausgabe der wahrscheinlichsten Obstruktionsart 01-04 bei der Schlafapnoe, wobei die jeweilige Obstruktionsart 01-04 aus einer dritten Gruppe von Klassen der Obstruktionsarten 01-04 stammt; alternativ kann der dritte Klassifikator K3 zur Klassifikation der jeweils wahrscheinlichsten Obstruktionsart 01-04 auch durch eine Parametereingabe vorbestimmt programmiert sein;
D) Bestimmen im jeweiligen Erkennungsmodus aus dem zu untersuchenden Schnarchgeräuschsignal Au durch den ersten Klassifikator K1 die Schnarchgeräuschentstehungsart S1-S4 , durch den zweiten Klassifikator K2 die Mundstellung M1-M2 und aus der bestimmten Schnarchgeräuschentstehungsart S1-S4 und Mundstellung M1-M2 durch den dritten Klassifikator K3 der daraus resultierenden Obstruktionsart 01-04; und
E) Ausgabe der zu dem untersuchenden Schnarchgeräuschsignal Au durch den ersten K1, den zweiten K2 und den dritten Klassifikator K3 bestimmten Obstruktionsart O1-O4 an einer Ausgabeschnittstelle 3.

In Fig. 3 ist ein Beispiel des oben dargelegten Verfahrens als Signalflussdiagramm dargestellt.

Bevorzugt weist das oben beschriebene Verfahren noch Folgendes auf, wobei das Trainieren des ersten Klassifikators K1 und das Trainieren des zweiten Klassifikators K2 mit einer Vielzahl der Schnarchgeräuschsignale Au separat voneinander erfolgen, wobei der erste Klassifikator K1 unabhängig von der Mundstellung M1-M2 und der zweite Klassifikator K2 unabhängig von der Schnarchgeräuschentstehungsart S1-S4 trainiert werden und lernen. Bevorzugt erfolgen das Trainieren bzw. das Lernen des ersten K1 und des zweiten Klassifikators K2 zeitversetzt oder zeitgleich.

Bevorzugt weist das oben beschriebene Verfahren auch folgendes auf, wobei das Trainieren des ersten Klassifikators K1 und das Trainieren des zweiten Klassifikators K2 mit einer weiteren Vielzahl der Schnarchgeräuschsignale Au gemeinsam und zeitgleich miteinander erfolgen, wobei dem dazu verwendeten jeweiligen Schnarchgeräuschsignal Au die jeweilige Schnarchgeräuschentstehungsart S1-S4 und die jeweilige Mundstellung M1-M2 zugeordnet sind.

Bevorzugt weist das oben beschriebene Verfahren auch folgendes auf, wobei im Erkennungsmodus durch den ersten Klassifikator K1 die jeweiligen Schnarchgeräuschentstehungsarten S1-S4 als jeweilige Wahrscheinlichkeitswerte bestimmt und dem dritten Klassifikator K3 zugeführt werden.

Bevorzugt weist das oben beschriebene Verfahren auch folgendes auf, wobei im Erkennungsmodus durch den zweiten Klassifikator K2 die jeweiligen Mundstellungen M1-M2 als jeweilige Wahrscheinlichkeitswerte bestimmt und dem dritten Klassifikator K3 zur Bestimmung der Obstruktionsart 01-04 zugeführt werden.

Bevorzugt weist das oben beschriebene Verfahren auch folgendes auf, wobei im Erkennungsmodus durch den dritten Klassifikator K3 aus den jeweiligen Schnarchgeräuschentstehungsarten S1-S4 und Mundstellungen M1-M2 die jeweilige Obstruktionsart 01-04 mit Angabe einer dazugehörigen jeweiligen Wahrscheinlichkeit bestimmt wird.

Bevorzugt weist das oben beschriebene Verfahren auch folgendes auf, wobei die erste Gruppe der Schnarchgeräuschentstehungsarten S1-S4 folgende Klassen umfasst: Velopharynx (V), Oropharynx (O), Zungengrundbereich (T), und/oder Kehldeckelbereich (E). Bevorzugt weist die jeweilige Schnarchgeräuschentstehungsart S1-S4 dabei eine Orientierung der Vibration auf, die beispielsweise eine laterale oder zirkuläre Vibration sein kann.

Bevorzugt umfasst die zweite Gruppe der Mundstellungen folgende Mundstellungen: Mund offen, Mund geschlossen. Alternativ bevorzugt kann die zweite Gruppe der Mundstellungen aber auch mehr als zwei Mundstellungen mit Zwischenmundstellungen aufweisen.

Bevorzugt ist das oben beschriebene Verfahren so ausgebildet, dass dem dritten Klassifikator K3 zusätzlich zur jeweiligen Schnarchgeräuschentstehungsart S1-S4 und der jeweiligen Mundstellung M1-M2 weitere den Schnarcher betreffende Schnarch- oder Patientendaten Px zugeführt werden, die vom dritten Klassifikator K3 beim Training und/oder bei der Erkennung der Obstruktionsart 01-04 berücksichtigt bzw. ausgewertet werden.

Bevorzugt umfassen die Schnarch- oder Patientendaten Px mindestens einen der folgenden Parameter: Bodymass-Index, Apnoe-Hypopnoe-Index, Tonsillengröße, Zungengröße, Friedman-Score, Uhrzeit des Schnarchens, Schlafdauer.

Insbesondere weist die Erfindung bevorzugt folgendes auf:
Punkt (1): Ein Klassifikationssystem (1) zu einer mikroprozessorgestützten Erkennung von Obstruktionsarten (01-04) bei einer Schlafapnoe durch entsprechende Klassifikation eines zu untersuchenden Schnarchgeräuschsignals (Au), umfassend:
   a) eine Eingangsschnittstelle für das jeweilige Schnarchgeräuschsignal (Au);
   b) einen ersten Klassifikator (K1), der ausgebildet ist, bei Eingabe einer ersten Vielzahl der Schnarchgeräuschsignale (Au) mit einer jeweils dazugehörigen Schnarchgeräuschentstehungsart (S1-S4) in einem Trainingsmodus so zu lernen, dass er in einem Erkennungsmodus zu dem jeweiligen Schnarchgeräuschsignal (Au) aus einer Gruppe von vorbestimmten Schnarchgeräuschentstehungsarten (S1-S4) die jeweils wahrscheinlichste Schnarchgeräuschentstehungsart (S1-S4) erkennt und ausgibt;
   c) einen zweiten Klassifikator (K2), der ausgebildet ist, bei Eingabe einer zweiten Vielzahl der Schnarchgeräuschsignale (Au) mit einer jeweils dazugehörigen Mundstellung (M1-M2) im dazugehörigen Trainingsmodus so zu lernen, dass er im Erkennungsmodus zu dem jeweiligen Schnarchgeräuschsignal (Au) aus einer Gruppe von vorbestimmten Mundstellungen (M1-M2) die jeweils wahrscheinlichste Mundstellung (M1-M2) erkennt und ausgibt;
   d) einen dritten Klassifikator (K3), der ausgebildet ist, in einem Erkennungsmodus bei Eingabe der vom ersten Klassifikator (K1) bestimmten Schnarchgeräuschentstehungsart (S1-S4), der vom zweiten Klassifikator (K2) bestimmten Mundstellung (M1-M2) aus einer Gruppe von vorbestimmten Obstruktionsarten (01-04) die wahrscheinlichste Obstruktionsart (01-04) zu erkennen und als Obstruktionsartsignal auszugeben; und
   e) eine Ausgabeschnittstelle (3) für das Obstruktionsartsignal zu einer Anzeige.
Punkt (2): Das Klassifikationssystem (1) gemäß Punkt (1), wobei der erste Klassifikator (K1) und der zweite Klassifikator (K2) so ausgebildet sind, dass das jeweilige Trainieren des ersten (K1) und des zweiten Klassifikators (K2) mit einer Vielzahl der Schnarchgeräuschsignale (Au) separat voneinander ausführbar ist, wobei der erste Klassifikator (K1) unabhängig von der Mundstellung (M1-M2) und der zweite Klassifikator (K2) unabhängig von der Schnarchgeräuschentstehungsart (S1-S4) trainiert und lernt.
Punkt (3): Das Klassifikationssystem (1) gemäß Punkt (1) oder Punkt (2), wobei der erste Klassifikator (K1) und der zweite Klassifikator (K2) so ausgebildet sind, dass das jeweilige Trainieren des ersten (K1) und des zweiten Klassifikators (K2) mit einer weiteren Vielzahl der Schnarchgeräuschsignale (Au) gemeinsam und zeitgleich miteinander erfolgt, wobei das dazu verwendete jeweilige Schnarchgeräuschsignal (Au) die jeweilige Schnarchgeräuschentstehungsart (S1-S4) und die jeweilige Mundstellung (M1-M2) als jeweilige Information dazu aufweist.
Punkt (4): Das Klassifikationssystem (1) gemäß einem oder mehreren der vorstehenden Punkte (1) - (3), wobei der erste Klassifikator (K1) ausgebildet ist, im Erkennungsmodus die jeweilige Schnarchgeräuschentstehungsart (S1-S4) mit einer jeweiligen Wahrscheinlichkeit zu bestimmen, zu indizieren und an den dritten Klassifikator (K3) weiterzuleiten.
Punkt (5): Das Klassifikationssystem (1) gemäß einem oder mehreren der Punkte (1) - (4), wobei der zweite Klassifikator (K2) ausgebildet ist, im Erkennungsmodus die jeweilige Mundstellung (M1-M2) mit einer jeweiligen Wahrscheinlichkeit zu bestimmen, zu indizieren und an den dritten Klassifikator (K3) weiterzuleiten.
Punkt (6): Das Klassifikationssystem (1) gemäß einem oder mehreren der Punkte (1) - (5), wobei der dritte Klassifikator (K3) ausgebildet ist, in einem Trainingsmodus bei Eingabe der vom ersten Klassifikator (K1) bestimmten Schnarchgeräusch-entstehungsart (S1-S4), der vom zweiten Klassifikator (K2) bestimmten Mundstellung (M1-M2) und einer Obstruktionsart (O1-O4) so zu lernen, dass er im Erkennungsmodus zu der jeweiligen Schnarchentstehungsart (S1-S4) und Mundstellung (M1-M2) die eingegebene Obstruktionsart (01-04) als die wahrscheinlichste Obstruktionsart (01-04) erkennt.
Punkt (7): Das Klassifikationssystem (1) gemäß einem oder mehreren der Punkte (1) - (6), wobei der dritte Klassifikator (K3) ausgebildet ist, in einem Erkennungsmodus die jeweilige Obstruktionsart (01-04) mit einer jeweiligen Wahrscheinlichkeit zu bestimmen, zu indizieren und an die Ausgabeschnittstelle (3) weiterzuleiten.
Punkt (8): Das Klassifikationssystem (1) gemäß einem oder mehreren der Punkte (1) - (7), wobei der dritte Klassifikator (K3) ausgebildet ist, zusätzlich zur jeweiligen Schnarchgeräuschentstehungsart (S1-S4) und der jeweiligen Mundstellung (M1-M2) weitere den Schnarcher betreffende Schnarch- oder Patientendaten (Px) über eine Eingabeschnittstelle (2) zu erfassen und im Trainingsmodus und/oder im Erkennungsmodus bei der Klassifikation der Obstruktionsart (01-04) als Parameter oder als Parametersignale zu berücksichtigen.
Punkt (9): Das Klassifikationssystem (1) gemäß Punkt (8), wobei die Schnarch- oder Patientendaten (Px) mindestens einen der folgenden Parameter umfasst: Bodymass-Index, Apnoe-Hypopnoe-Index, Tonsillengröße, Zungengröße, Friedman-Score, Uhrzeit des Schnarchens, Schlafdauer.
Punkt (10): Das Klassifikationssystem (1) gemäß einem oder mehreren der Punkte (1) - (9), wobei der erste Klassifikator (K1) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression; und/oder
   wobei der zweite Klassifikator (K2) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression; und/oder
   wobei der dritte Klassifikator (K3) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression; oder
   der dritte Klassifikator (K3) auf einer Matrixwahrscheinlichkeitsberechnung eines ersten Eingangsvektors aus den Schnarchgeräuschentstehungsarten (S1-S4) und aus mindestens einem weiteren zweiten Eingangsvektor der Mundstellungen (M1-M2) basiert, deren summarische Wahrscheinlichkeiten die verschiedenen Obstruktionsarten (01-04) mit deren Wahrscheinlichkeiten ergeben.

Zur Klarheit sei angemerkt, dass unbestimmte Artikel in Verbindung mit einem Gegenstand oder Zahlenangaben, wie beispielsweise "ein" Gegenstand den Gegenstand nicht zahlenmäßig auf genau einen Gegenstand begrenzt, sondern dass damit mindestens "ein" Gegenstand gemeint ist. Dies gilt für alle unbestimmten Artikel wie beispielsweise "ein", "eine" usw.

Zur Klarheit sollen die Ausdrücke "erste/r", "zweite/r", "dritte/r" usw. hierin nur dazu verwendet werden, verschiedene Vielzahlen, Elemente und/oder Komponenten voneinander zu unterscheiden. Daher kann beispielsweise eine erste Vielzahl auch als die zweite Vielzahl und dementsprechend die zweite Vielzahl als die erste Vielzahl bezeichnet werden, ohne von den Lehren der vorliegenden Erfindung abzuweichen.

Es versteht sich, dass anstelle der hierin aufgezählten zwei oder vier Klassen der Schnarchgeräuschentstehungsarten, der Mundstellungen und der Obstruktionsarten auch andere jeweilige Vielzahlen verwendet oder detektiert werden können.

Die In den Ansprüchen genannten Bezugszeichen dienen nur der besseren Verständlichkeit und beschränken die Ansprüche in keiner Weise auf die in den Figuren dargestellten Formen.

### Bezugszeichenliste

- 1: Klassifikationssystem
- 2: Eingabeschnittstelle
- 3: Ausgabeschnittstelle
- Au: Schnarchgeräuschsignal
- Sx, S1-S4: Schnarchgeräuschentstehungsart
- Mx, M1, M2: Mundstellung
- Ox, 01-04: Obstruktionsart
- K1: erster Klassifikator
- K2: zweiter Klassifikator
- K3: dritter Klassifikator
- Px: Schnarch- oder Patientendaten
- V: Velopharynx
- O: Oropharynx
- T: Bereich des Zungengrundes
- E: Bereich des Kehldeckels

## Patentansprüche

1. Klassifikationssystem (1) zu einer mikroprozessorgestützten Erkennung von Obstruktionsarten (01-04) bei einer Schlafapnoe durch entsprechende Klassifikation eines zu untersuchenden Schnarchgeräuschsignals (Au), umfassend:
a) eine Eingangsschnittstelle für das jeweilige Schnarchgeräuschsignal (Au);
b) einen ersten Klassifikator (K1), der ausgebildet ist, bei Eingabe einer ersten Vielzahl der Schnarchgeräuschsignale (Au) mit einer jeweils dazugehörigen Schnarchgeräuschentstehungsart (S1-S4) in einem Trainingsmodus so zu lernen, dass er in einem Erkennungsmodus zu dem jeweiligen Schnarchgeräuschsignal (Au) aus einer Gruppe von vorbestimmten Schnarchgeräuschentstehungsarten (S1-S4) die jeweils wahrscheinlichste Schnarchgeräuschentstehungsart (S1-S4) erkennt und ausgibt;
c) einen zweiten Klassifikator (K2), der ausgebildet ist, bei Eingabe einer zweiten Vielzahl der Schnarchgeräuschsignale (Au) mit einer jeweils dazugehörigen Mundstellung (M1-M2) im dazugehörigen Trainingsmodus so zu lernen, dass er im Erkennungsmodus zu dem jeweiligen Schnarchgeräuschsignal (Au) aus einer Gruppe von vorbestimmten Mundstellungen (M1-M2) die jeweils wahrscheinlichste Mundstellung (M1-M2) erkennt und ausgibt;
d) einen dritten Klassifikator (K3), der ausgebildet ist, in einem Erkennungsmodus bei Eingabe der vom ersten Klassifikator (K1) bestimmten Schnarchgeräuschentstehungsart (S1-S4), der vom zweiten Klassifikator (K2) bestimmten Mundstellung (M1-M2) aus einer Gruppe von vorbestimmten Obstruktionsarten (01-04) die wahrscheinlichste Obstruktionsart (01-04) zu erkennen und als Obstruktionsartsignal auszugeben;
e) wobei der dritte Klassifikator (K3) ausgebildet ist, zusätzlich zur jeweiligen Schnarchgeräuschentstehungsart (S1-S4) und der jeweiligen Mundstellung (M1-M2) weitere den Schnarcher betreffende Schnarch- oder Patientendaten (Px) über eine Eingabeschnittstelle (2) zu erfassen und im Trainingsmodus und/oder im Erkennungsmodus bei der Klassifikation der Obstruktionsart (01-04) als Parameter oder als Parametersignale zu berücksichtigen; und
f) eine Ausgabeschnittstelle (3) für das Obstruktionsartsignal zu einer Anzeige.

2. Das Klassifikationssystem (1) gemäß Anspruch 1, wobei der erste Klassifikator (K1) und der zweite Klassifikator (K2) so ausgebildet sind, dass das jeweilige Trainieren des ersten (K1) und des zweiten Klassifikators (K2) mit einer Vielzahl der Schnarchgeräuschsignale (Au) separat voneinander ausführbar ist, wobei der erste Klassifikator (K1) unabhängig von der Mundstellung (M1-M2) und der zweite Klassifikator (K2) unabhängig von der Schnarchgeräuschentstehungsart (S1-S4) trainiert und lernt.

3. Das Klassifikationssystem (1) gemäß Anspruch 1 oder 2, wobei der erste Klassifikator (K1) und der zweite Klassifikator (K2) so ausgebildet sind, dass das jeweilige Trainieren des ersten (K1) und des zweiten Klassifikators (K2) mit einer weiteren Vielzahl der Schnarchgeräuschsignale (Au) gemeinsam und zeitgleich miteinander erfolgt, wobei das dazu verwendete jeweilige Schnarchgeräuschsignal (Au) die jeweilige Schnarchgeräuschentstehungsart (S1-S4) und die jeweilige Mundstellung (M1-M2) als jeweilige Information dazu aufweist.

4. Das Klassifikationssystem (1) gemäß einem oder mehreren der vorstehenden Ansprüche, wobei der erste Klassifikator (K1) ausgebildet ist, im Erkennungsmodus die jeweilige Schnarchgeräuschentstehungsart (S1-S4) mit einer jeweiligen Wahrscheinlichkeit zu bestimmen, zu indizieren und an den dritten Klassifikator (K3) weiterzuleiten.

5. Das Klassifikationssystem (1) gemäß einem oder mehreren der vorstehenden Ansprüche, wobei der zweite Klassifikator (K2) ausgebildet ist, im Erkennungsmodus die jeweilige Mundstellung (M1-M2) mit einer jeweiligen Wahrscheinlichkeit zu bestimmen, zu indizieren und an den dritten Klassifikator (K3) weiterzuleiten.

6. Das Klassifikationssystem (1) gemäß einem oder mehreren der vorstehenden Ansprüche, wobei der dritte Klassifikator (K3) ausgebildet ist, in einem Trainingsmodus bei Eingabe der vom ersten Klassifikator (K1) bestimmten Schnarchgeräuschentstehungsart (S1-S4), der vom zweiten Klassifikator (K2) bestimmten Mundstellung (M1-M2) und einer Obstruktionsart (01-04) so zu lernen, dass er im Erkennungsmodus zu der jeweiligen Schnarchentstehungsart (S1-S4) und Mundstellung (M1-M2) die eingegebene Obstruktionsart (01-04) als die wahrscheinlichste Obstruktionsart (O1-04) erkennt.

7. Das Klassifikationssystem (1) gemäß einem oder mehreren der vorstehenden Ansprüche, wobei der dritte Klassifikator (K3) ausgebildet ist, im Erkennungsmodus die jeweilige Obstruktionsart (01-04) mit einer jeweiligen Wahrscheinlichkeit zu bestimmen, zu indizieren und an die Ausgabeschnittstelle (3) weiterzuleiten.

8. Das Klassifikationssystem (1) gemäß einem oder mehreren der vorstehenden Ansprüche, wobei die Schnarch- oder Patientendaten (Px) mindestens einen der folgenden Parameter umfasst: Bodymass-Index, Apnoe-Hypopnoe-Index, Tonsillengröße, Zungengröße, Friedman-Score, Uhrzeit des Schnarchens, Schlafdauer.

9. Das Klassifikationssystem (1) gemäß einem oder mehreren der vorstehenden Ansprüche, wobei der erste Klassifikator (K1) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression.

10. Das Klassifikationssystem (1) gemäß einem oder mehreren der vorstehenden Ansprüche, wobei der zweite Klassifikator (K2) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression.

11. Das Klassifikationssystem (1) gemäß einem oder mehreren der vorstehenden Ansprüche, wobei der dritte Klassifikator (K3) auf einem der folgenden Verfahren des maschinellen Lernens basiert: Stützvektormethode (Support Vector Machine, SVM), Naive-Bayes-System, Least Mean Square Verfahren, k-Nearest Neighbours Verfahren (k-NN), Linear Discriminant Analysis (LDA), Random Forests Verfahren (RF), Extreme Learning Machine (ELM), Multilayer Perceptron (MLP), Deep Neural Network (DNN), logistische Regression.

12. Das Klassifikationssystem (1) gemäß einem der vorstehenden Ansprüche 1 - 10, wobei der dritte Klassifikator (K3) auf einer Matrixwahrscheinlichkeitsberechnung eines ersten Eingangsvektors aus den Schnarchgeräuschentstehungsarten (S1-S4) und aus mindestens einem weiteren zweiten Eingangsvektor der Mundstellungen (M1-M2) basiert, deren summarische Wahrscheinlichkeiten die verschiedenen Obstruktionsarten (01-04) mit deren Wahrscheinlichkeiten ergeben.
